(19) Europäisches Patentamt
European Patent Office
Office européen des brevets

(11) Veröffentlichungsnummer: **0 016 985**
**A1**

(12) **EUROPÄISCHE PATENTANMELDUNG**

(21) Anmeldenummer: **80101084.4**

(22) Anmeldetag: **04.03.80**

(51) Int. Cl.³: **C 07 D 303/48,** C 07 D 307/32, A 01 N 43/08, A 01 N 43/20

(30) Priorität: **16.03.79 CH 2492/79**
**31.07.79 CH 7079/79**
**18.01.80 CH 426/80**

(43) Veröffentlichungstag der Anmeldung: **15.10.80**
**Patentblatt 80/21**

(84) Benannte Vertragsstaaten: **AT BE CH DE FR GB IT LU NL SE**

(71) Anmelder: **F. HOFFMANN-LA ROCHE & CO.**
**Aktiengesellschaft, CH-4002 Basel (CH)**

(72) Erfinder: **Dorn, Franz, Dr., Bohnackerstrasse 5,**
**CH-8157 Dielsdorf (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

(54) **Substituierte Anilide, Verfahren zu deren Herstellung, fungicide Mittel enthaltend solche Verbindungen sowie Verwendung solcher Verbindungen bzw. Mittel zur Bekämpfung von Pflanzenfungi.**

(57) Die Erfindung betrifft substituierte Anilide der allgemeinen Formel

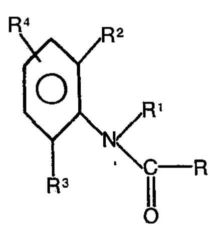

worin R eine der Gruppen

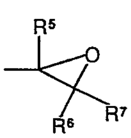 (a) und 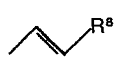 (b)

EP 0 016 985 A1

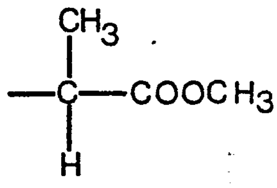 (c) und 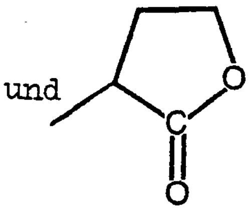 (d)

$R^2$ und $R^3$ jeweils Alkyl mit 1 bis 3 Kohlenstoffatomen, Alkoxy mit 1 bis 3 Kohlenstoffatomen oder Halogen, $R^4$ Wasserstoff, Alkyl mit 1 bis 3 Kohlenstoffatomen oder Halogen, $R^5$, $R^6$ und $R^7$ jeweils Wasserstoff, Methyl oder Aethyl und $R^8$ Methyl oder Aethyl bedeuten, mit den Massgaben:

(i) dass die Gesamtzahl der Kohlenstoffatome in den Substituenten $R^2$, $R^3$ und $R^4$ 6 nicht übersteigt,

und

(ii) dass, wenn R eine Gruppe (b) bedeutet, $R^1$ nur die Gruppe (d) bedeuten kann.

Die Verbindungen der Formel I besitzen fungicide Eigenschaften und eignen sich als fungicide Mittel.

Die Erfindung betrifft ferner ein Verfahren zur Herstellung von Verbindungen der Formel I, Verbindungen der Formel I als fungicide Mittel, fungicide Mittel, die mindestens eine Verbindung der Formel I als Wirkstoff enthalten sowie die Verwendung solcher fungicider Mittel zur Bekämpfung von Pilzen an Pflanzen.

Unter Alkyl und als Alkylteil einer Alkoxygruppe in der Formel I sind je nach Zahl der angegebenen Kohlenstoffatome folgende Gruppen zu verstehen: Methyl, Aethyl, n-Propyl und Isopropyl.

0016985

F.Hoffmann-La Roche & Co. Aktiengesellschaft,Basel,Schweiz

RAN 6103/9k

# Substituierte Anilide, Verfahren zu deren Herstellung, fungicide Mittel enthaltend solche Verbindungen sowie Verwendung solcher Verbindungen bzw. Mittel zur Bekämpfung von Pflanzenfungi.

Die Erfindung betrifft Verbindungen der allgemeinen Formel

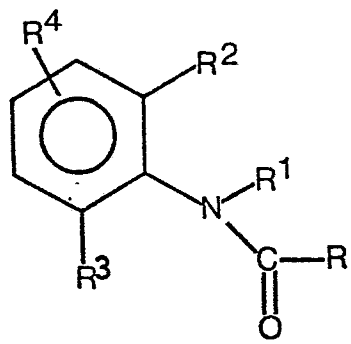

I

worin R eine der Gruppen

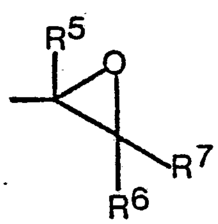 (a) und 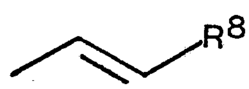 (b)

$R^1$ eine der Gruppen

Pa/ 16.1.80

Der Ausdruck Halogen umfasst Fluor, Chlor, Brom und Jod.

Hat $R^2$ oder $R^3$ die Bedeutung Alkyl mit 1 bis 3 Kohlenstoffatomen, so ist dies vorzugsweise Methyl.

$R^4$ bedeutet vorzugsweise Wasserstoff.

Die Phenylgruppe der Verbindungen der Formel I ist vorzugsweise 2,6-disubstituiert, insbesondere 2,6-Dichlorphenyl, 2-Chlor-6-methylphenyl oder 2,6-Dimethylphenyl.

Eine interessante Untergruppe von Verbindungen der Formel I besteht aus solchen Verbindungen, worin R eine Gruppe (a) und $R^3$ Alkyl mit 1 bis 3 Kohlenstoffatomen oder Halogen bedeuten und $R^1$, $R^2$ und $R^4$ die oben angegebenen Bedeutungen besitzen.

Eine weitere interessante Untergruppe von Verbindungen der Formel I besteht aus solchen Verbindungen, worin R eine Gruppe (b) und $R^1$ die Gruppe (d) bedeuten und $R^2$, $R^3$ und $R^4$ die oben angegebenen Bedeutungen besitzen.

Ganz besonders bevorzugte Verbindungen sind:

N-(2,3-Epoxybutyryl)-N-(2,6-xylyl)alanin-methylester,
N-(2,3-Epoxy-2-methylpropionyl)-N-(2,6-xylyl)alaninmethylester,
N-(Tetrahydro-2-oxo-3-furyl)-2',6'-crotonoxylidid und 2',6'-Dimethyl-N-(tetrahydro-2-oxo-3-furyl)-2-pentenanilid.

In den Verbindungen der Formel I sind asymmetrische Kohlenstoffatome vorhanden, so dass die Verbindungen als optische Antipoden vorliegen können. Unabhängig davon kommt auch in gewissen Fällen eine

Atropisomerie vor. Durch das Vorliegen der Doppelbindung in den Verbindungen der Formel I, worin R eine Gruppe (b) bedeutet, tritt für diese Verbindungen zusätzlich geometrische Isomerie auf. Die Formel I soll demnach all diese möglichen isomeren Formen umfassen.

Das erfindungsgemässe Verfahren zur Herstellung der Verbindungen der Formel I ist dadurch gekennzeichnet, dass man

a) eine Verbindung der Formel

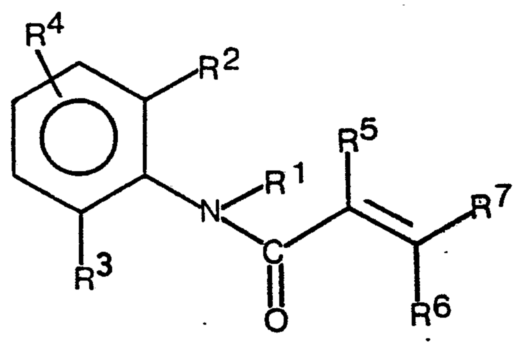

II

worin $R^1$, $R^2$, $R^3$, $R^4$, $R^5$, $R^6$ und $R^7$ die oben angegebenen Bedeutungen besitzen,

epoxidiert,

b) eine Verbindung der Formel

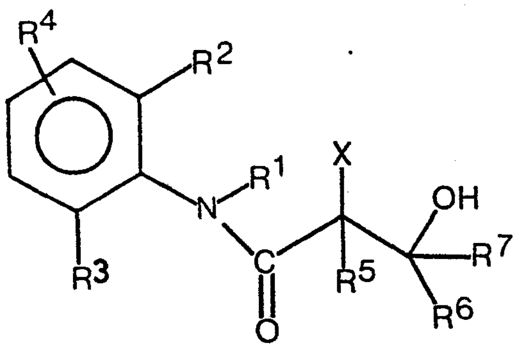

III

worin $R^1$, $R^2$, $R^3$, $R^4$, $R^5$, $R^6$ und $R^7$ die oben angegebenen Bedeutungen besitzen und X Chlor oder Brom bedeutet,

mit einer Base behandelt, um den entsprechenden Halogenwasserstoff HX zu eliminieren,

oder

c) eine Verbindung der Formel

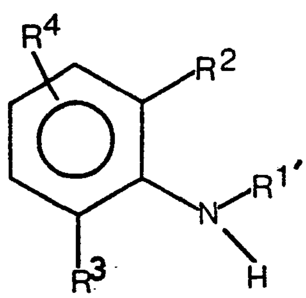

IV

worin $R^{1'}$ die Gruppe (d) und $R^2$, $R^3$ und $R^4$ die oben angegebenen Bedeutungen besitzen,

mit einer Carbonsäure der Formel

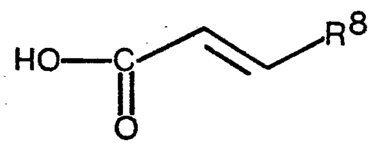

V

worin $R^8$ die oben angegebene Bedeutung besitzt, oder einem reaktionsfähigen Säurehalogenid, Säureanhydrid, Ester oder Amid, vorzugsweise mit einem Säurehalogenid oder dem Säureanhydrid, davon, umsetzt.

Bei der Verfahrensvariante a) kann die Epoxidierung der Verbindungen der Formel II zweckmässig in der Weise durchgeführt werden, dass man die betreffende Verbindung in einem inerten Lösungsmittel, insbesondere in einem halogenierten Kohlenwasserstoff, wie Methylenchlorid oder Chloroform, löst und in einem zwischen 10° und der Rückflusstemperatur des Reaktionsgemisches liegenden Temperaturbereich mit einer organischen Persäure, z.B. mit Peressigsäure, Perbenzoesäure, m-Chlorperbenzoesäure, Perphthalsäure oder mit Wasserstoffperoxid in beispielsweise Methanol oder Aethanol unter Zugabe einer äquivalenten Menge Natriumhydroxid, behandelt.

Die Verfahrensvariante b) kann zweckmässigerweise durchgeführt werden, indem man das Ausgangsmaterial der Formel III, gelöst in einem inerten Lösungsmittel, z.B. Aceton oder Toluol, in einem Temperaturbereich zwischen 0°C und 50°C, insbesondere bei Raumtemperatur, mit einer Base, vorzugsweise einer in organischen Lösungsmitteln nicht löslichen Base, wie Natriumcarbonat oder Kaliumcarbonat, behandelt.

Die Umsetzung nach der Verfahrensvariante c) kann in An- oder Abwesenheit von gegenüber den Reaktionsteilnehmern inerten Lösungs- oder Verdünnungsmitteln durchgeführt werden, z.B. aliphatischen oder aromatischen Kohlenwasserstoffen wie Benzol, Toluol, Xylolen oder Petroläthern; halogenierten Kohlenwasserstoffen wie Chlorbenzolen, Methylenchlorid, Aethylenchlorid oder Chloroform; Aethern und ätherartigen Verbindungen wie Dialkyläthern, Dioxan oder Tetrahydrofuran; Nitrilen wie Acetonitril; N,N-dialkylierten Amiden wie Dimethylformamid; Dimethylsulfoxid; Ketonen wie Methyläthylketon; und Gemischen solcher Lösungsmittel untereinander.

Die Reaktionstemperaturen liegen zwischen 0 und 180°C, vorzugsweise zwischen 20 und 120°C. In manchen Fällen ist die Verwendung von säurebindenden Mitteln bzw. Kondensationsmitteln vorteilhaft. Als solche kommen tertiäre Amine wie Trialkylamine (z.B. Triäthylamin), Pyridin und Alkylpyridine, oder anorganische Basen, wie die Oxide und Hydroxide, Hydrogencarbonate und Carbonate von Alkali- und Erdalkalimetallen sowie Natriumacetat in Betracht. Als säurebindendes Mittel kann ausserdem ein Ueberschuss des jeweiligen Anilinderivates der Formel IV dienen.

Als Säurehalogenide der Verbindungen der Formel V sind die Säurechloride oder Säurebromide bevorzugt.

Einige der in der Verfahrensvariante a) verwendbaren Ausgangsmaterialien der Formel II, d.h. diejenigen der Formel II, worin $R^1$ die Gruppe (d), $R^5$ und $R^6$ Wasserstoff und $R^7$ Methyl oder Aethyl bedeuten, entsprechen einem Teil der Verbindungen der Formel I, d.h. denjenigen der Formel I, worin R eine Gruppe (b) bedeutet. Die restlichen Ausgangsmaterialien der Formel II können analog zur Verfahrensvariante c) hergestellt werden, indem man eine entsprechende Verbindung der Formel

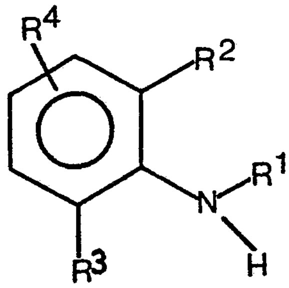

IV'

worin $R^1$, $R^2$, $R^3$ und $R^4$ die oben angegebenen Bedeutungen besitzen,

mit einer Carbonsäure der Formel

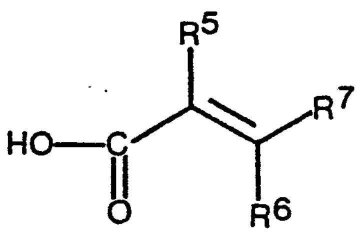

V'

worin $R^5$, $R^6$ und $R^7$ die oben angegebenen Bedeutungen besitzen,

oder einem reaktionsfähigen Säurehalogenid, Säureanhydrid, Ester oder Amid, vorzugsweise mit einem Säurehalogenid, insbesondere dem Säurechlorid oder Säurebromid, oder dem Säureanhydrid dieser Verbindung der Formel V' umsetzt.

Die Verbindungen der Formel III können hergestellt werden, indem man eine Verbindung der Formel II mit unterchloriger Säure bzw. unterbromiger Säure behandelt. Zweckmässigerweise kann die unterbromige Säure in situ erzeugt werden, z.B. indem man N-Bromsuccinimid oder N-Bromacetamid in wässrig-organischer Lösung

verwendet. Als der organische Teil dieser wässrig-organischen Lösung kommen mit Wasser mischbare organische Lösungsmittel in Frage, insbesondere Dimethoxyäthan oder Tetrahydrofuran. Die Reaktion wird geeigneterweise in einem Temperaturbereich zwischen $0^{o}C$ und $50^{o}C$, insbesondere bei Raumtemperatur, durchgeführt.

Diese Methode und die anschliessende Reaktion zwischen der so hergestellten Verbindung der Formel III und einer Base nach der Verfahrensvariante b) sind im Artikel von E.E. van Tamelen et al., J.A.C.S. 85, 3296 (1963) generell beschrieben.

Die Verbindungen der Formel IV können hergestellt werden, indem man eine Verbindung der Formel

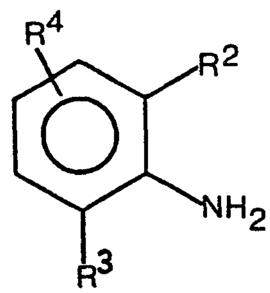

VI

worin $R^2$, $R^3$ und $R^4$ die oben angegebenen Bedeutungen besitzen,

mit einer Verbindung der Formel

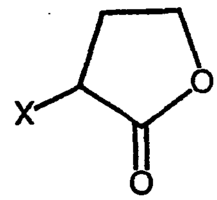

VII

worin X Chlor, Brom, Tosyloxy oder Mesyloxy bedeutet,
umsetzt.

Die Umsetzung der Verbindungen der Formel VI mit der Verbindung der Formel VII kann in An- oder Abwesenheit von gegenüber den Reaktionsteilnehmern inerten Lösungs- oder Verdünnungsmitteln durchgeführt werden, wie beispielsweise den obenerwähnten Mitteln, die für die Reaktion zwischen den Verbindungen der Formeln IV und V geeignet sind.

Die Reaktionstemperaturen liegen zweckmässig zwischen 0°C und 180°C, vorzugsweise zwischen 20°C und 120°C. In manchen Fällen ist die Verwendung von säurebindenden Mitteln bzw. Kondensationsmitteln vorteilhaft. Als solche kommen bevorzugt anorganische Basen, wie die Oxide und Hydroxide, Hydrogencarbonate und Carbonate von Alkali- und Erdalkalimetallen sowie Natriumacetat in Betracht. Als säurebindendes Mittel kann ausserdem ein Ueberschuss des jeweiligen Anilinderivates der Formel VI dienen.

Die als Ausgangsmaterialien verwendbaren Verbindungen der Formel V bzw. V', Verbindungen der Formel IV', worin $R^1$ eine Gruppe (c) bedeutet, und Verbindungen der Formeln VI und VII sind entweder bekannt oder können nach an sich bekannten Methoden hergestellt werden.

Im allgemeinen ist die Verfahrensvariante a) bevorzugt für die Herstellung derjenigen Verbindungen der Formel I, worin $R^1$ die Gruppe (c) bedeutet, während sich die Verfahrensvariante b) für die Herstellung derjenigen Verbindungen der Formel I, worin $R^1$ die Gruppe (d) bedeutet, besser eignet.

Sofern keine gezielte Synthese zur Isolierung reiner Isomerer durchgeführt wird, fällt normalerweise ein Produkt als Gemisch zweier oder mehrerer Isomerer an. Die Isomeren können nach bekannten Methoden aufgetrennt werden.

Die Verbindungen der Formel I zeigen auf Pflanzen, die von Pilzen befallen sind, eine spezifische fungicide Wirkung. Als Pflanzen können im Rahmen vorliegender Erfindung beispielsweise Mais, Gemüse, Zuckerrüben, Soja, Erdnüsse, Obstbäume, Zierpflanzen, vor allem aber Reben, Hopfen, Gurkengewächse (Gurken, Kürbis, Melonen), Solanaceen wie Kartoffeln, Tabak und Tomaten, sowie auch Bananen-, Ananas-, Avocado-, Kakao- und Naturkautschuk-Gewächse genannt werden.

Die Verbindungen der Formel I, worin R eine Gruppe (a) bedeutet, sind ferner wirksam gegen Eier von beispielsweise Spinnentieren und eignen sich somit zur Einarbeitung als Wirkstoffe auch in ovicide Mittel.

Mit den Wirkstoffen der Formel I können an Pflanzen oder Pflanzenteilen (Früchte, Blüten, Laubwerk, Stengel, Knollen, Wurzeln) dieser und verwandter Nutzkulturen die auftretenden Pilze eingedämmt oder vernichtet werden, wobei auch später zuwachsende Pflanzenteile von derartigen Pilzen verschont bleiben. Die Wirkstoffe sind gegen die den folgenden Klassen angehörenden phytopathogenen Pilze wirksam: Basidiomycetes wie vor allem Rostpilze;

dann aber besonders gegen Phycomyceten, insbesondere der zur Klasse der Oomycetes gehörenden Gattungen Phytophthora, Peronospora, Pseudoperonospora, Pythium, Bremia oder Plasmopara. Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Wie aus dem nachstehenden biologischen Beispiel hervorgeht, wirken die Verbindungen der Formel I unter Gewächshausbedingungen bereits bei einer Konzentration von 5 mg bis 500 mg Wirksubstanz pro Liter Spritzbrühe. Im Freiland werden vorteilhaft Konzentrationen von 100 g bis 2500 g Wirksubstanz der Formel I pro Hektar und Behandlung zur Anwendung gebracht. Beispielsweise wird zur erfolgreichen Rebmehltaubekämpfung eine Konzentration von 200 g bis 1000 g, vorzugsweise 200 g bis 600 g Wirksubstanz pro Hektar und Anwendung mit Vorteil benützt. Zur Getreiderostbekämpfung werden vorzugsweise Konzentrationen von 500 g bis 2500 g, besonders bevorzugt hinsichtlich der wirksamsten Vertreter 500 g bis 2000 g Wirksubstanz pro Hektar und Anwendung eingesetzt.

Ein Teil der Verbindungen der Formel I zeichnet sich durch hohe systemische Wirkungsentfaltung aus.

Durch Sekundärverteilung der Wirksubstanz (Gasphasenwirkung) können zusätzlich nicht behandelte Pflanzenteile geschützt werden.

Für praktische Zwecke können die Verbindungen der Formel I als für Wirbeltiere weitgehend ungiftig qualifiziert werden. Einzelne Vertreter zeigen $LD_{50}$-Werte an der Maus zwischen 400 und 1000 mg pro kg Körpergewicht, andere Vertreter zeigen $LD_{50}$-Werte, die zwischen 1000 und 10'000 mg pro kg Körpergewicht im akuten Toxizitätstest an der Maus liegen.

Die Verbindungen der Formel I können in verschiedenartigen Mitteln, z.B. Spritzbrühen, wässrigen Suspensionen, Emulsionen, emulgierbaren Konzentraten, pulverförmigen Präparaten, formuliert werden. Im allgemeinen enthält ein Mittel, je nach dessen Art, zwischen 0,0001 und 95 Gewichtsprozent Verbindung bzw. Verbindungen der Formel I als Wirkstoff.

Zur Herstellung von pulverförmigen Präparaten kommen verschiedene inerte pulverförmige Trägerstoffe in Frage, wie z.B. Kaolin, Bentonit, Talkum, Schlämmkreide, Magnesiumcarbonat oder Kieselgur. Die aktiven Komponenten werden mit solchen Trägerstoffen vermischt, z.B. durch Zusammenmahlen; oder man imprägniert den inerten Trägerstoff mit einer Lösung der aktiven Komponenten und entfernt dann das Lösungsmittel durch Abdunsten, Erhitzen oder durch Absaugen unter vermindertem Druck. Solche pulverförmige Präparate können als Stäubemittel mit Hilfe der üblichen Verstäubergeräte auf die zu schützenden Pflanzen aufgebracht werden. Durch Zusatz von Netz- und/oder Dispergiermitteln kann man solche pulverförmige Präparate mit Wasser leicht benetzbar machen, so dass sie in Form von wässrigen Suspensionen als Spritzmittel anwendbar sind.

Zur Herstellung emulgierbarer Konzentrate können die aktiven Stoffe beispielsweise mit einem Emulgiermittel gemischt oder auch in einem inerten Lösungsmittel gelöst und mit einem Emulgator gemischt werden. Durch Verdünnen solcher Konzentrate mit Wasser erhält man gebrauchsfertige Emulsionen. Derartige Konzentrate können 5-95 Gewichtsprozent, insbesondere 25-75 Gewichtsprozent, Wirkstoff enthalten.

Die erfindungsgemässen fungiciden Mittel können neben den Wirkstoffen der Formel I auch andere Wirkstoffe enthalten, z.B. anderweitige fungicide Mittel, insekticide und

acaricide Mittel, Pflanzenwachstumsregulatoren und Düngemittel. Solche Kombinationsmittel eignen sich zur Verbreiterung des Wirkungsspektrums oder zur spezifischen Beeinflussung des Pflanzenwachstums, z.B. durch Reduktion der Anfälligkeit gegen Oidium- und Botrytis-Arten im Rebbau, und können in Form von z.B. pulverförmigen Präparaten oder Spritzbrühen je nach Einsatzgebiet vorliegen. Beispiele solcher bekannten Wirkstoffe sind in der folgenden Tabelle zusammengefasst:

| Stoffklasse | Vertreter |
|---|---|
| Dithiocarbamate | Mancozeb (Gemisch von [(1,2-Aethandiylbis(carbamodithioato))(2-)]mangan und [(1,2-Aethandiylbis(carbamodithioato))(2-)]zink |
| Kupferverbindungen | Basisches $CuCl_2$ |
| Bordeaux-Brühe | |
| Schwefel | |
| Phthalimide | Folpet(N-(Trichlormethansulfenyl)-phthalimid) |
| | Captafol (3a,4,7,7a-Tetrahydro-N-(1,1,2,2-tetrachloräthansulfenyl)-phthalimid) |
| Triphenylzinnverbindungen | Fentinhydroxid (Triphenylzinnhydroxid)<br>Fentinacetat (Triphenylzinnacetat) |

| | |
|---|---|
| Benzimidazol-2-carbamidsäure-methylester-Bildner | Benomyl (Methyl-2[1-(butylcarbamoyl) benzimidazolyl]carbamat) |
| | Thiophanate (1,2-Di-(3-äthoxycarbonyl-2-thioureido)benzol) |
| Dicarboximide | Iprodion (3-(3,5-Dichlorphenyl)-1-isopropylcarbamoylhydantoin) |
| | Procymidon (N-(3',5'-Dichlorphenyl)-1,2-dimethylcyclopropan-1,2-dicarboximid) |
| | Vinclozoline (3-(3,5-Dichlorphenyl)-5-methyl-5-vinyl-1,3-oxazolidin-2,4-dion) |
| Nitrile | Chlorothalonil (2,4,5,6-Tetrachlor-1,3-dicyanbenzol) |
| Phosphorsäureester | |
| Carbamate | |
| Pyrethroide | |
| Insektenwuchsregulatoren | |
| Gibberelline | |
| Düngemittel | auf Stickstoff-, Phosphor-, Kalium-, Calzium- und Spurenelement-Basis. |

Die nachfolgenden Beispiele dienen zur näheren Erläuterung der Erfindung. Die Temperaturangaben beziehen sich auf Celsiusgrade. Sofern nicht anders vermerkt, ist bei der Nennung eines Wirkstoffs der Formel I stets das

anfallende racemische Isomerengemisch gemeint.

I. Herstellung der Wirkstoffe:

Beispiel 1

Zu 11,2 g N-Acryloyl-N-2,6-xylylalanin-methylester in 200 ml eines 1:1-Gemisches von Methanol und Aethanol werden nacheinander 10 g 30%ige wässerige Natronlauge und 6,6 g 30%iges wässeriges Wasserstoffperoxid gegeben. Das Reaktionsgemisch wird 3 Stunden bei 40°C gerührt, dann mit Eiswasser versetzt und mit Methylenchlorid extrahiert. Die organische Phase wird mit wässeriger Natriumbicarbonatlösung gewaschen, über Natriumsulfat getrocknet und das Lösungsmittel abgedampft. Chromatographie des so erhaltenen Rohproduktes an Silicagel mit einem Gemisch von Hexan und Essigester lieferte reinen N-(2,3-Epoxy-propionyl)-N-(2,6-xylyl)alanin-methylester als farbloses Oel, $n_D^{20}$ : 1,5238.

Beispiel 2

Zu 5 g N-(2-Methylacryloyl)-N-2,6-xylylalanin-methylester, gelöst in 40 ml Chloroform, werden 8 g 40%ige Peressigsäure gegeben und anschliessend 24 Stunden unter Rückfluss erhitzt. Dann wird mit Eiswasser versetzt, mit Methylenchlorid extrahiert, die organische Phase mit wässeriger Natriumbicarbonatlösung gewaschen, über Natriumsulfat getrocknet und das Lösungsmittel abgedampft. Umkristallisation des Rohproduktes aus Essigester/Hexan liefert reinen N-(2,3-Epoxy-2-methylpropionyl)-N-(2,6-xylyl)alanin-methylester. Fp.: 110-114°C.

In analoger Weise erhält man:

- aus N-Crotonyl-N-2,6-xylylalanin-methylester und 40%iger Peressigsäure den N-(2,3-Epoxybutyryl)-N-(2,6-xylyl)alanin-methylester (Diastereomerengemisch) als farbloses Oel, beim Stehenlassen teilweise kris-

tallisierend, $n_D^{20}$ : 1,5130

- aus N-(2-Methylcrotonyl)-N-2,6-xylylalanin-methylester und 40%iger Peressigsäure, nach Chromatographie des Rohproduktes an Silicagel, den N-(2,3-Epoxy-2-methylbutyryl)-N-(2,6-xylyl)alanin-methylester als farbloses Oel, $n_D^{20}$ : 1,5037

- aus N-(3-Methylcrotonyl)-N-(2,6-xylyl)alanin-methylester und 40%iger Peressigsäure, nach Chromatographie des Rohproduktes an Silicagel, den N-(2,3-Epoxy-3-methylbutyryl)-N-(2,6-xylyl)alanin-methylester als farbloses Oel, $n_D^{20}$ : 1,5102

## Beispiel 3

4 g N-(Tetrahydro-2-oxo-3-furyl)-2',6'-crotonoxylidid werden in 60 ml Dimethoxyäthan-Wasser 2:1 gelöst und bei 10° mit 7,8 g N-Bromsuccinimid versetzt. Dann wird 24 Stunden bei Raumtemperatur gerührt, anschliessend auf Wasser gegossen, mit Methylenchlorid extrahiert, über Natriumsulfat getrocknet, filtriert und eingedampft. Das so erhaltene Reaktionsprodukt wird in 60 ml Aceton gelöst, mit 10 g wasserfreiem Kaliumcarbonat versetzt und über Nacht bei Raumtemperatur gerührt. Dann wird mit Aether verdünnt, vom Kaliumcarbonat filtriert und eingedampft. Das so erhaltene Oel wird an Silicagel chromatographiert. Man erhält zwei isomere Formen von N-(Tetrahydro-2-oxo-3-furyl)-2,3-epoxy-2',6'-crotonoxylidid, die weniger polare als zähes Oel, die polarere kristallin mit Smp. 144-145°.

## Beispiel 4

12 g 3-[N-(2,6-Dimethylphenyl)]-amino-tetrahydro-2-furanon werden in 50 ml Toluol gelöst und nacheinander 9 g wasserfreies Natriumcarbonat und 7 g Crotonsäurechlorid zugesetzt. Dann wird über Nacht bei Raumtemperatur gerührt, anschliessend auf Eiswasser gegossen und mit Diäthyläther extrahiert. Die organische Phase wird mit 2 n Salzsäure und gesättigter Kochsalzlösung gewaschen, über Natriumsulfat getrocknet, filtriert und eingedampft. Das so erhaltene Rohprodukt wird aus Essigester/Hexan umkristallisiert. Man erhält N-(Tetrahydro-2-oxo-3-furyl)-2',6'-crotonoxylidid. Fp.: 119-121°.

In analoger Weise erhält man:

- Aus 3-[N-(2,6-Dimethylphenyl)]-amino-tetrahydro-2-furanon und 2-Pentensäurechlorid das 2',6'-Dimethyl-N-(tetrahydro-2-oxo-3-furyl)-2-pentenanilid. Fp.: 110-111°.

II. Biologische Beispiele:

## Beispiel 5

TETRANYCHUS OVIZID

Testorganismus: Tetranychus urticae, gemeine Spinnmilbe

Testmethode:

Buschbohnen-Blattrondellen (Ø 25 mm) werden mit adulten Weibchen infiziert. 1 Tag später werden die Weibchen entfernt und die Blattrondellen mit ca. 30-50 Eiern werden mit acetonischer Wirkstofflösung besprüht. Die behandelten Blattrondellen werden auf feuchten Schaumstoff gelegt und bei 25° und 60% relativer Feuchtigkeit inkubiert. Unbehandelte und mit Aceton behandelte Rondellen dienen als Kontrollparzellen. Testdauer: 6 Tage. Die Resultate werden ausgedrückt als prozentuale Reduktion der Ueberlebensrate im Vergleich zu den Kontrollparzellen. Die Resultate sind in nachstehender Tabelle I zusammenge-

fasst.

Tabelle I

| Dosierung ($10^{-x}$g AS/$cm^2$): | 5 | 6 |
|---|---|---|
| | %Wirkung | |
| Stand der Technik (RIDOMIL): | | |
| N-(Methoxyacetyl)-N-(2,6-xylyl) alanin-methylester | 0 | 0 |
| Erfindungsgemässe Verbindungen | | |
| N-(2,3-Epoxypropionyl)-N-(2,6-xylyl)alanin-methylester | 100 | 0 |
| N-(2,3-Epoxybutyryl)-N-(2,6-xylyl)alanin-methylester | 100 | 0 |
| N-(2,3-Epoxy-2-methylbutyryl)-N-(2,6-xylyl)alanin-methylester | 100 | 38 |
| N-(2,3-Epoxy-2-methylpropionyl)-N-(2,6-xylyl)alanin-methylester | 100 | 0 |
| N-(2,3-Epoxy-3-methylbutyryl)-N-(2,6-xylyl)alanin-methylester | 100 | 2 |

(Unbehandelte Kontrollen: 3% Mortalität)
AS = Wirkstoff

## Beispiel 6

PERONOSPORA VITICOLA

Testmethode:

2 Rebstecklinge der Sorte Riesling x Sylvaner wurden jeweils im 4-5 Blattstadium mit einer wässerigen Dispersion der Testsubstanz (wie allgemein üblich aufbereitet als Spritzpulver) allseitig gründlich besprüht und anschliessend in einer Klimakabine bei 17°C, 70-80% relativer Luftfeuchtigkeit und einer Photoperiode von 16 Stunden weiterkultiviert. Nach 2 Tagen erfolgte die Infektion der Versuchspflanzen durch Besprühen der Blattunterseiten mit in destilliertem Wasser suspendierten Zoosporangien (ca. 300'000 Sporangien/ml) von Peronospora viticola. Danach wurden die Rebpflanzen wie folgt inkubiert: 2 Tage bei 18°C und Taupunktbedingungen im Dunkeln und 5 Tage bei 20°C, einer relativen Luftfeuchtigkeit von 70-80% und einer Photoperiode von 18 Stunden. Um die Fruktifikation von Peronospora viticola zu induzieren, wirde am 8. Tag nach der Infektion die relative Luftfeuchtigkeit auf über 90% erhöht.

Die Versuchsauswertung erfolgte jeweils am 9. Tag nach der Infektion durch Ermittlung der durch Peronospora viticola befallenen Blattfläche in % gegenüber der infizierten, nicht behandelten Kontrolle. Die Resultate sind in nachstehender Tabelle II zusammengefasst.

Tabelle II

| Erfindungsgemässe Verbindungen | Konzentration (in mg/l Spritzbrühe) | Wirkung (in %) |
|---|---|---|
| N-(2,3-Epoxypropionyl)-N-(2,6-xylyl)alanin-methylester | 50<br>16<br>5 | 100<br>90<br>70 |
| N-(2,3-Epoxybutyryl)-N-(2,6-xylyl)alanin-methylester | 50<br>16<br>5 | 100<br>97<br>40 |
| N-(2,3-Epoxy-2-methylpropionyl)-N-(2,6-xylyl)alanin-methylester | 50<br>16<br>5 | 100<br>100<br>100 |
| N-(2,3-Epoxy-3-methylbutyryl)-N-(2,6-xylyl)alanin-methylester | 50<br>16<br>5 | 100<br>75<br>75 |
| N-(Tetrahydro-2-oxo-3-furyl)-2',6'-crotonoxylidid | 50<br>16<br>5 | 100<br>100<br>80 |
| Stand der Technik | | |
| N-(Tetrahydro-2-oxo-3-furyl)-2',6'-chloracetanilid | 50<br>16<br>5 | 100<br>70<br>40 |

## Beispiel 7

### PYTHIUM DEBARYANUM

Testmethode:

Mit Wasserstoffperoxid desinfizierte Zuckerrübensamen der Sorte "Kawemono" werden 1 cm tief in gedämpfte und mit Pythium debaryanum kontaminierte Erde ausgesät (15 Samen je Plastiktopf mit 100 ml Inhalt). Die Erdmischung besteht aus 2 Teilen gedämpfter Landerde, 1 Teil sterilisierten Sandes und 5 Gew.-% einer einwöchigen Pythium debaryanum-Kultur inklusiv Nährsubstrat. Die Applikation erfolgt unmittelbar nach der Aussaat durch Angiessen des Bodens, wobei 25 und 6 mg der Testsubstanz je Liter Erdmischung ausgebracht werden.

Die Inkubation der Aussaaten erfolgt unter kontrollierten Bedingungen in Klimakabinen bei 21-23°, 80% relativer Luftfeuchtigkeit und Langtagbedingungen (240 $W/m^2$, 16 Stunden/Tag).

Am 12. Tag nach der Aussaat werden die durch Pythium debaryanum erkrankten Sämlinge gezählt, und die Wirkung gegenüber infizierten Kontrollparzellen wird in % nach Abbott ermittelt.

Tabelle III

| Erfindungsgemässe Verbindung | Konzentration (in mg/l Spritzbrühe) | Wirkung (in %) |
|---|---|---|
| N-(Tetrahydro-2-oxo-3-furyl)-2',6'-crotonoxylidid | 25<br>6 | 100<br>100 |
| N-(2,3-Epoxybutyryl)-N-(2,6-xylyl)alanin-methylester | 25<br>6 | 100<br>100 |
| N-(2,3-Epoxy-2-methylpropionyl)-N-(2,6-xylyl)alanin-methyl-ester | 25<br>6 | 100<br>100 |

III. Formulierungsbeispiele:

Beispiel 8

| | | |
|---|---|---|
| Wirkstoff der Formel I (flüssig) | 750 | Gew.-Teile |
| Ricinusöl-Aethylenoxid-Addukt | 100 | " |
| Ca-Dodecylbenzolsulfonat | 25 | " |
| Aromatisches Lösungsmittel (Gemisch von $C_{10}$-Alkylbenzolen) zu | 1000 | Vol.-Teilen |

Die Komponenten werden im Lösungsmittel gelöst. Das entstandene emulgierbare Konzentrat wird zur Herstellung der gebrauchsfertigen Spritzbrühen in Wasser gegeben, wobei eine für Stunden stabile Emulsion (Oel/Wasser) entsteht.

Beispiel 9

| | | |
|---|---|---|
| Wirkstoff der Formel I (fest) | 80 | Gew.-Teile |
| Kieselsäure, hydratisiert | 10 | " |
| Na-Laurylsulfat | 1 | " |
| Na-Lignosulfonat | 2 | " |
| Kaolin (Kaolinit) | 7 | " |

Die Komponenten werden in einer Mischmaschine gemischt, diese Mischung in einer Luftstrahlmühle gemahlen und hernach wieder gemischt. Das entstandene Spritzpulver, in welchem der Wirkstoff in einer Partikelgrösse von unter 10 Mikron vorliegt, lässt sich mit Wasser gut benetzen und ergibt eine gut schwebefähige Dispersion zur Behandlung der Pflanzen.

0016985

DS 6103/9k

Patentansprüche

1. Verbindungen der allgemeinen Formel

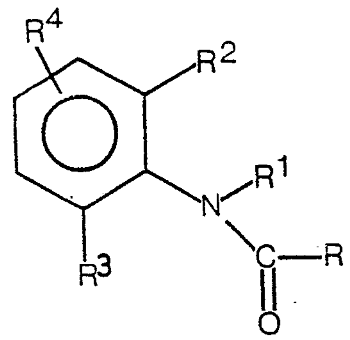

I

worin R eine der Gruppen

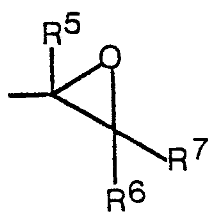

(a) und

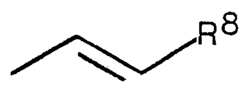

(b)

$R^1$ eine der Gruppen

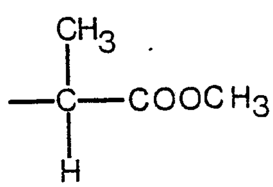

(c) und

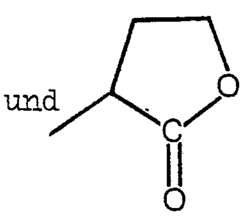

(d)

$R^2$ und $R^3$ jeweils Alkyl mit 1 bis 3 Kohlenstoffatomen, Alkoxy mit 1 bis 3 Kohlenstoffatomen oder Halogen, $R^4$ Wasserstoff, Alkyl mit 1 bis 3 Kohlenstoffatomen oder Halogen, $R^5$, $R^6$ und $R^7$ jeweils Wasserstoff, Methyl oder Aethyl, und $R^8$ Methyl oder Aethyl bedeuten,

mit den Massgaben:

(i) dass die Gesamtzahl der Kohlenstoffatome in den Substituenten $R^2$, $R^3$ und $R^4$ 6 nicht übersteigt,

und

(ii) dass, wenn R eine Gruppe (b) bedeutet, $R^1$ nur die Gruppe (d) bedeuten kann.

2. Verbindungen nach Anspruch 1, worin R eine Gruppe (a) und $R^3$ Alkyl mit 1 bis 3 Kohlenstoffatomen oder Halogen bedeuten.

3. Verbindungen nach Anspruch 1, worin R eine Gruppe (b) und $R^1$ eine Gruppe (d) bedeuten.

4. Verbindungen nach einem der Ansprüche 1 bis 3, worin $R^4$ Wasserstoff bedeutet.

5. Verbindungen nach Anspruch 4, worin der Phenylrest 2,6-Dichlorphenyl, 2-Chlor-6-methyl-phenyl oder 2,6-Dimethylphenyl bedeutet.

6. N-(2,3-Epoxybutyryl)-N-(2,6-xylyl)alanin-methylester.

7. N-(2,3-Epoxy-2-methylpropionyl)-N-(2,6-xylyl)-alanin-methylester.

8. N-(Tetrahydro-2-oxo-3-furyl)-2',6'-crotonoxylidid.

9. 2',6'-Dimethyl-N-(tetrahydro-2-oxo-3-furyl)-2-pentenanilid.

10. N-(Tetrahydro-2-oxo-3-furyl)-2,3-epoxy-2',6'-crotonoxylidid.

11. Eine Verbindung nach Anspruch 1 ausgewählt unter:

N-(2,3-Epoxypropionyl)-N-(2,6-xylyl)alanin-methylester,

N-(2,3-Epoxy-2-methylbutyryl)-N-(2,6-xylyl)-alanin-methylester,

und N-(2,3-Epoxy-3-methylbutyryl)-N-(2,6-xylyl)-alanin-methylester.

0016985
DS 6103/9k

12. Verbindungen nach einem der Ansprüche 1 bis 11 als fungicide Mittel.

13. Fungicides Mittel, dadurch gekennzeichnet, dass es eine wirksame Menge von mindestens einer Verbindung der allgemeinen Formel

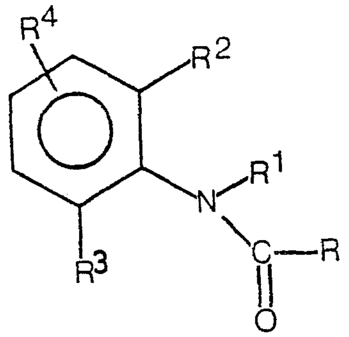

I

worin R eine der Gruppen

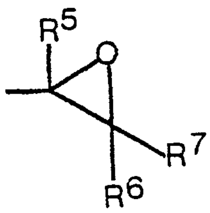 (a) und 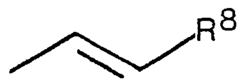 (b)

$R^1$ eine der Gruppen

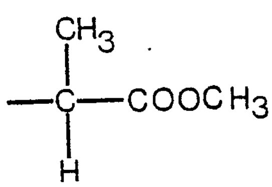 (c) und 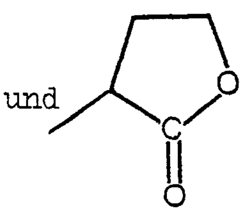 (d)

$R^2$ und $R^3$ jeweils Alkyl mit 1 bis 3 Kohlenstoffatomen, Alkoxy mit 1 bis 3 Kohlenstoffatomen oder Halogen, $R^4$ Wasserstoff, Alkyl mit 1 bis 3 Kohlenstoffatomen oder Halogen, $R^5$, $R^6$ und $R^7$ jeweils Wasserstoff, Methyl oder Aethyl, und $R^8$ Methyl oder Aethyl bedeuten,

mit den Massgaben:

(i) dass die Gesamtzahl der Kohlenstoffatome in den Substituenten $R^2$, $R^3$ und $R^4$ 6 nicht übersteigt,

und

(ii) dass, wenn R eine Gruppe (b) bedeutet, $R^1$ nur die Gruppe (d) bedeuten kann,

sowie inertes Trägermaterial enthält.

14. Fungicides Mittel nach Anspruch 13, dadurch gekennzeichnet, dass es eine wirksame Menge von N-(2,3-Epoxybutyryl)-N-(2,6-xylyl)alanin-methylester sowie inertes Trägermaterial enthält.

15. Fungicides Mittel nach Anspruch 13, dadurch gekennzeichnet, dass es eine wirksame Menge von N-(2,3-Epoxy-2-methylpropionyl)-N-(2,6-xylyl)alanin-methylester sowie inertes Trägermaterial enthält.

16. Fungicides Mittel nach Anspruch 13, dadurch gekennzeichnet, dass es eine wirksame Menge von N-(Tetrahydro-2-oxo-3-furyl)-2',6'-crotonoxylidid sowie inertes Trägermaterial enthält.

17. Fungicides Mittel nach Anspruch 13, dadurch gekennzeichnet, dass es eine wirksame Menge von 2',6'-Dimethyl-N-(tetrahydro-2-oxo-3-furyl)-2-pentenanilid sowie inertes Trägermaterial enthält.

0016985
DS 6103/9k

18. Verfahren zur Herstellung der Verbindungen der allgemeinen Formel

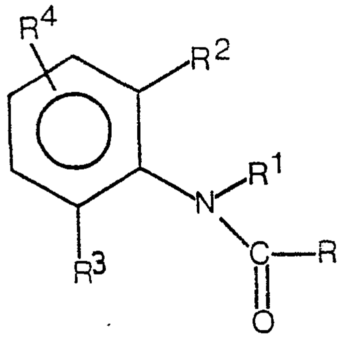

I

worin R eine der Gruppen

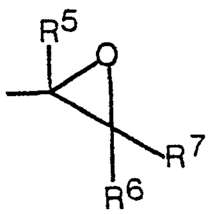 (a) und 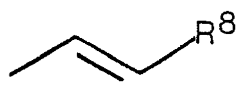 (b)

$R^1$ eine der Gruppen

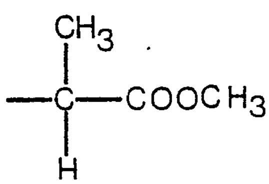 (c) und 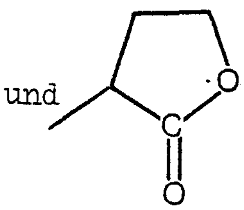 (d)

$R^2$ und $R^3$ jeweils Alkyl mit 1 bis 3 Kohlenstoffatomen, Alkoxy mit 1 bis 3 Kohlenstoffatomen oder Halogen, $R^4$ Wasserstoff, Alkyl mit 1 bis 3 Kohlenstoffatomen oder Halogen, $R^5$, $R^6$ und $R^7$ jeweils Wasserstoff, Methyl oder Aethyl, und $R^8$ Methyl oder Aethyl bedeuten,

mit den Massgaben:

(i) dass die Gesamtzahl der Kohlenstoffatome in den Substituenten $R^2$, $R^3$ und $R^4$ 6 nicht übersteigt,

und

(ii) dass, wenn R eine Gruppe (b) bedeutet, $R^1$ nur die Gruppe (d) bedeuten kann, dadurch gekennzeichnet, dass man

a) eine Verbindung der Formel

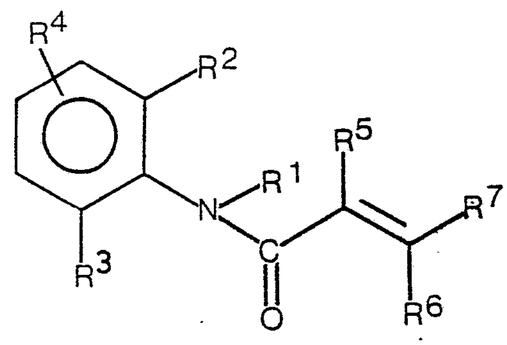

II

worin $R^1$, $R^2$, $R^3$, $R^4$, $R^5$, $R^6$ und $R^7$ die oben angegebenen Bedeutungen besitzen, epoxidiert,

b) eine Verbindung der Formel

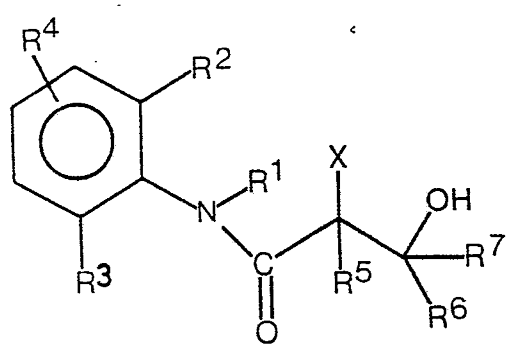

III

worin $R^1$, $R^2$, $R^3$, $R^4$, $R^5$, $R^6$ und $R^7$ die oben angegebenen Bedeutungen besitzen, und X Chlor oder Brom bedeutet,

0016985
DS 6103/9k

mit einer Base behandelt, um den entsprechenden Halogenwasserstoff HX zu eliminieren,

oder

c) eine Verbindung der Formel

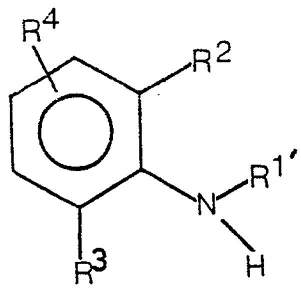

IV

worin $R^{1'}$ die Gruppe (d) und $R^2$, $R^3$ und $R^4$ die oben angegebenen Bedeutungen besitzen,
mit einer Carbonsäure der Formel

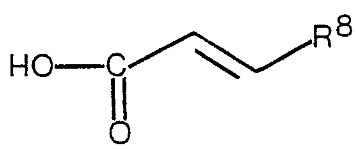

V

worin $R^8$ die oben angegebene Bedeutung besitzt,
oder einem reaktionsfähigen Säurehalogenid, Säureanhydrid, Ester oder Amid davon umsetzt.

19. Verfahren nach Anspruch 18, worin $R^2$ und $R^3$ Methyl und $R^4$ Wasserstoff bedeuten.

20. Verfahren nach Anspruch 19 zur Herstellung von N-(2,3-Epoxybutyryl)-N-(2,6-xylyl)alanin-methylester, dadurch gekennzeichnet, dass man N-Crotonyl-N-(2,6-xylyl)-alanin-methylester epoxidiert.

0016985

DS 6103/9k

21. Verfahren zur Bekämpfung von Pflanzenfungi, dadurch gekennzeichnet, dass man die zu schützenden Pflanzen mit einer wirksamen Menge einer in den Ansprüchen 1 bis 11 genannten Verbindung bzw. eines in den Ansprüchen 13 bis 17 genannten Mittels behandelt.

0016985



22. Verwendung einer der in den Ansprüchen 1 bis 11 genannten Verbindungen bzw. eines der in den Ansprüchen 13 bis 17 genannten Mittel zur Bekämpfung von Pflanzenfungi.

***

 0016985

DP 6103/9k

## Patentansprüche für Oesterreich

1. Fungicides Mittel, dadurch gekennzeichnet, dass es eine wirksame Menge von mindestens einer Verbindung der allgemeinen Formel

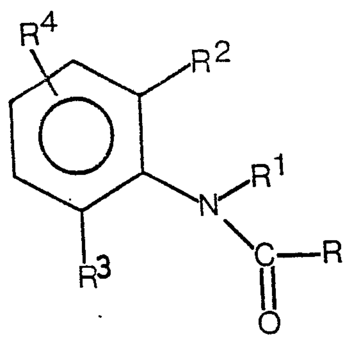

I

worin R eine der Gruppen

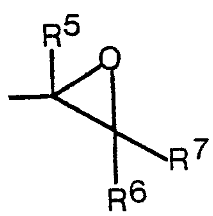 (a) und 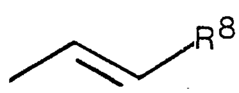 (b)

$R^1$ eine der Gruppen

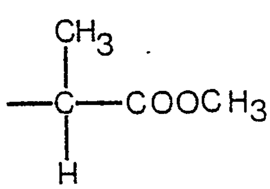 (c) und 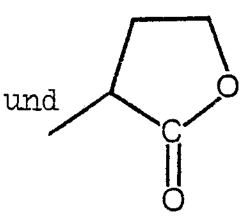 (d)

$R^2$ und $R^3$ jeweils Alkyl mit 1 bis 3 Kohlenstoffatomen, Alkoxy mit 1 bis 3 Kohlenstoffatomen oder Halogen, $R^4$ Wasserstoff, Alkyl mit 1 bis 3 Kohlenstoffatomen oder Halogen, $R^5$, $R^6$ und $R^7$ jeweils

Wasserstoff, Methyl oder Aethyl, und $R^8$ Methyl oder Aethyl bedeuten,

mit den Massgaben:

(i) dass die Gesamtzahl der Kohlenstoffatome in den Substituenten $R^2$, $R^3$ und $R^4$ 6 nicht übersteigt,

und

(ii) dass, wenn R eine Gruppe (b) bedeutet, $R^1$ nur die Gruppe (d) bedeuten kann,

sowie inertes Trägermaterial enthält.

2. Fungicides Mittel nach Anspruch 1, worin in der Formel I R eine Gruppe (a) und $R^3$ Alkyl mit 1 bis 3 Kohlenstoffatomen oder Halogen bedeuten.

3. Fungicides Mittel nach Anspruch 1, worin in der Formel I R eine Gruppe (b) und $R^1$ die Gruppe (d) bedeuten.

4. Fungicides Mittel nach Anspruch 1, worin $R^4$ in der Formel I Wasserstoff bedeutet.

5. Fungicides Mittel nach Anspruch 2, worin $R^4$ in der Formel I Wasserstoff bedeutet.

6. Fungicides Mittel nach Anspruch 3, worin $R^4$ in der Formel I Wasserstoff bedeutet.

7. Fungicides Mittel nach Anspruch 4, worin der Phenylrest in der Formel I 2,6-Dichlorphenyl, 2-Chlor-6-methyl-phenyl oder 2,6-Dimethylphenyl bedeutet.

8. Fungicides Mittel, dadurch gekennzeichnet, dass es eine wirksame Menge von N-(2,3-Epoxybutyryl)-N-(2,6-xylyl)alanin-methylester sowie inertes Trägermaterial enthält.

9. Fungicides Mittel, dadurch gekennzeichnet, dass es eine wirksame Menge von N-(2,3-Epoxy-2-methylpropionyl)-N-(2,6-xylyl)-alanin-methylester sowie inertes Trägermaterial enthält.

10. Fungicides Mittel, dadurch gekennzeichnet, dass es eine wirksame Menge von N-(Tetrahydro-2-oxo-3-furyl)-2',6'-crotonoxylidid sowie inertes Trägermaterial enthält.

11. Fungicides Mittel, dadurch gekennzeichnet, dass es eine wirksame Menge von 2',6'-Dimethyl-N-(tetrahydro-2-oxo-3-furyl)-2-pentenanilid sowie inertes Trägermaterial enthält.

12. Fungicides Mittel, dadurch gekennzeichnet, dass es eine wirksame Menge von N-(Tetrahydro-2-oxo-3-furyl)-2,3-epoxy-2',6'-crotonoxylidid sowie inertes Trägermaterial enthält.

13. Fungicides Mittel, dadurch gekennzeichnet, dass es eine wirksame Menge von einer Verbindung ausgewählt unter:

N-(2,3-Epoxypropionyl)-N-(2,6-xylyl)alanin-methylester,

N-(2,3-Epoxy-2-methylbutyryl)-N-(2,6-xylyl)-alanin-methylester,

und N-(2,3-Epoxy-3-methylbutyryl)-N-(2,6-xylyl)-alanin-methylester,

sowie inertes Trägermaterial enthält.

14. Verfahren zur Herstellung der Verbindungen der allgemeinen Formel

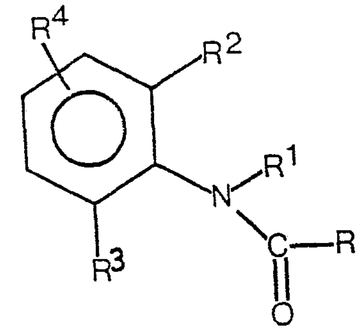

I

worin R eine der Gruppen

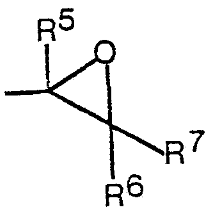 (a) und 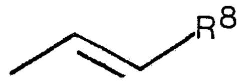 (b)

$R^1$ eine der Gruppen

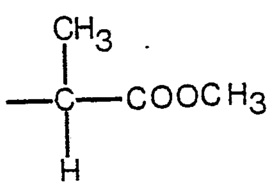 (c) und 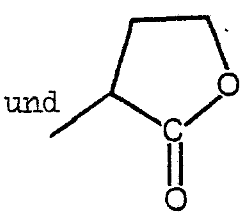 (d)

$R^2$ und $R^3$ jeweils Alkyl mit 1 bis 3 Kohlenstoffatomen, Alkoxy mit 1 bis 3 Kohlenstoffatomen oder Halogen, $R^4$ Wasserstoff, Alkyl mit 1 bis 3 Kohlenstoffatomen oder Halogen, $R^5$, $R^6$ und $R^7$ jeweils Wasserstoff, Methyl oder Aethyl, und $R^8$ Methyl oder Aethyl bedeuten,

mit den Massgaben:

(i) dass die Gesamtzahl der Kohlenstoffatome in den Substituenten $R^2$, $R^3$ und $R^4$ 6 nicht übersteigt,

und

(ii) dass, wenn R eine Gruppe (b) bedeutet, $R^1$ nur die Gruppe (d) bedeuten kann, dadurch gekennzeichnet, dass man

a) eine Verbindung der Formel

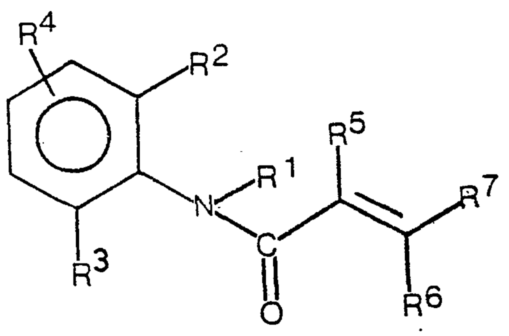

II

worin $R^1$, $R^2$, $R^3$, $R^4$, $R^5$, $R^6$ und $R^7$ die oben angegebenen Bedeutungen besitzen, epoxidiert,

b) eine Verbindung der Formel

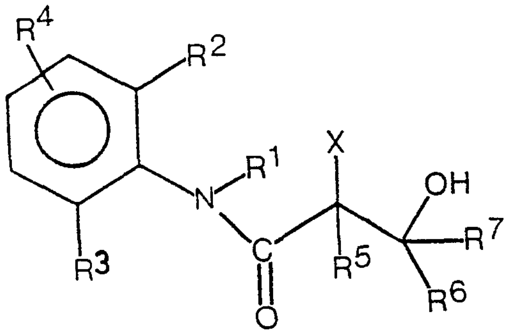

III

worin $R^1$, $R^2$, $R^3$, $R^4$, $R^5$, $R^6$ und $R^7$ die oben angegebenen Bedeutungen besitzen, und X Chlor oder Brom bedeutet,
mit einer Base behandelt, um den entsprechenden Halogenwasserstoff HX zu eliminieren,

oder

c) eine Verbindung der Formel

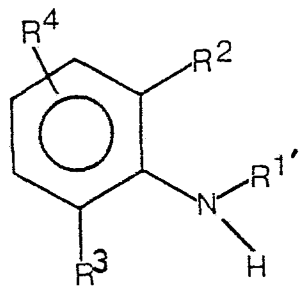

IV

worin $R^{1'}$ die Gruppe (d) und $R^2$, $R^3$ und $R^4$ die oben angegebenen Bedeutungen besitzen,
mit einer Carbonsäure der Formel

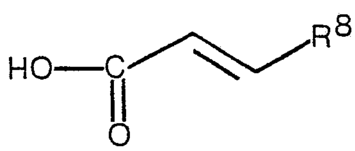

V

worin $R^8$ die oben angegebene Bedeutung besitzt, oder einem reaktionsfähigen Säurehalogenid, Säureanhydrid, Ester oder Amid davon umsetzt.

15. Verfahren nach Anspruch 14 zur Herstellung von Verbindungen der Formel I, worin R eine Gruppe (a) und $R^3$ Alkyl mit 1 bis 3 Kohlenstoffatomen oder Halogen bedeuten, dadurch gekennzeichnet, dass es gemäss der Verfahrensvariante a) durchgeführt wird.

16. Verfahren nach Anspruch 14 zur Herstellung von Verbindungen der Formel I, worin R eine Gruppe (a) bedeutet, dadurch gekennzeichnet, dass es gemäss der Verfahrensvariante b) durchgeführt wird.

17. Verfahren nach Anspruch 14 zur Herstellung von Verbindungen der Formel I, worin R eine Gruppe (b) und $R^1$ die Gruppe (d) bedeuten, dadurch gekennzeichnet, dass es gemäss der Verfahrensvariante c) durchgeführt wird.

18. Verfahren nach Anspruch 14, worin $R^2$ und $R^3$ Methyl und $R^4$ Wasserstoff bedeuten.

19. Verfahren zur Herstellung von N-(2,3-Epoxybutyryl)-N-(2,6-xylyl)alanin-methylester, dadurch gekennzeichnet, dass man N-Crotonyl-N-(2,6-xylyl)-alanin-methylester epoxidiert.

20. Verfahren zur Herstellung eines fungiciden Mittels, dadurch gekennzeichnet, dass man mindestens eine der in den Ansprüchen 1, 4, 7 und 12 genannten Verbindungen mit einem inerten Trägermaterial vermischt.

21. Verfahren zur Herstellung eines fungiciden Mittels, dadurch gekennzeichnet, dass man mindestens eine der in den Ansprüchen 2, 5, 8, 9 und 13 genannten Verbindungen mit einem inerten Trägermaterial vermischt.

22. Verfahren zur Herstellung eines fungiciden Mittels, dadurch gekennzeichnet, dass man mindestens eine der in den Ansprüchen 3, 6, 10 und 11 genannten Verbindungen mit einem inerten Trägermaterial vermischt.

0016985

DP 6103/9k

23. Verfahren zur Bekämpfung von Pflanzenfungi, dadurch gekennzeichnet, dass man die zu schützenden Pflanzen mit einer wirksamen Menge eines der in den Ansprüchen 1, 4, 7 und 12 genannten Mittel behandelt.

24. Verfahren zur Bekämpfung von Pflanzenfungi, dadurch gekennzeichnet, dass man die zu schützenden Pflanzen mit einer wirksamen Menge eines der in den Ansprüchen 2, 5, 8, 9 und 13 genannten Mittel behandelt.

25. Verfahren zur Bekämpfung von Pflanzenfungi, dadurch gekennzeichnet, dass man die zu schützenden Pflanzen mit einer wirksamen Menge eines der in den Ansprüchen 3, 6, 10 und 11 genannten Mittel behandelt.

0016985

DP 6103/9k

26. Verwendung eines der in den Ansprüchen 1, 4, 7 und 12 genannten Mittel zur Bekämpfung von Pflanzenfungi.

27. Verwendung eines der in den Ansprüchen 2, 5, 8, 9 und 13 genannten Mittel zur Bekämpfung von Pflanzenfungi.

28. Verwendung eines der in den Ansprüchen 3, 6, 10 und 11 genannten Mittel zur Bekämpfung von Pflanzenfungi.

***

Europäisches Patentamt

# EUROPÄISCHER RECHERCHENBERICHT

0016985

Nummer der Anmeldung

EP 80 10 1084.4

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| | DE - A1 - 2 804 299 (CIBA-GEIGY)<br>* Ansprüche 1, 15 *<br>-- | 13,18 | C 07 D 303/48<br>C 07 D 307/32<br>A 01 N 43/08<br>A 01 N 43/20 |
| | DE - A1 - 2 724 785 (SCHERING)<br>* Ansprüche 47 bis 49 *<br>-- | 18 | |
| | DE - A1 - 2 724 786 (SCHERING)<br>* Ansprüche 36, 37 *<br>-- | 18 | RECHERCHIERTE SACHGEBIETE (Int. Cl.³) |
| P | DE - A1 - 2 847 075 (CHEVRON RESEARCH)<br>* Anspruch 1 *<br>---- | 18 | A 01 N 43/00<br>C 07 D 303/48<br>C 07 D 307/32<br>C 07 D 407/12 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 16-06-1980 | FROELICH |